# FASCICULE DE BREVET EUROPEEN

(11) **EP 1 221 931 B1**
(45) Date de publication et mention de la délivrance du brevet: **27.09.2006**
(21) Numéro de dépôt: 00964327.1
(22) Date de dépôt: 20.09.2000
(51) Int. Cl.: A61K 8/04, A61K 8/19, A61K 8/25, A61K 8/26, A61Q 5/06

(54) **COMPOSITION COSMETIQUE COMPRENANT DES NANOPARTICULES CONTENANT DE L'ALUMINE**
KOSMETISCHE ZUSAMMENSETZUNGEN MIT ALUMINIUMOXID ENTHALTENDEN NANOPARTIKELN
COSMETIC COMPOSITION COMPRISING NANOPARTICLES CONTAINING ALUMINA

(30) Priorité: 30.09.1999 FR 9912236
(43) Date de publication de la demande: 17.07.2002
(73) Titulaire: L'ORÉAL, 75008 Paris (FR)
(72) Inventeur: GIROUD, Franck, F-92110 Clichy (FR); SAMAIN, Henri, F-91570 Bièvres (FR)
(74) Mandataire: Bourdeau, Françoise
(86) Numéro de dépôt international: PCT/FR2000/002604
(87) Numéro de publication internationale: WO 2001/022926

(56) Documents cités:
- EP-A- 0 682 939
- EP-A- 0 965 324
- WO-A-97/38667
- DE-A- 19 520 237
- US-A- 3 819 827
- US-A- 5 830 509
- DATABASE WPI Week 198330 Derwent Publications Ltd., London, GB; AN 1983-720285 XP002140159 & JP 58 103301 A (TOYO AEROSOL KOGYO KK)

## Description

L'invention a pour objet des compositions cosmétiques, notamment capillaires, comprenant des nanoparticules contenant de l'alumine. Elle vise également l'utilisation de nanoparticules contenant de l'alumine dans des compositions cosmétiques capillaires pour donner du gonflant aux cheveux.

Au sens de la présente invention, on entend par *« composition pour donner du gonflant aux cheveux »*, une composition qui a pour effet d'augmenter le volume global de la coiffure, en donnant aux cheveux de la vigueur et du ressort. On entend par *« composition de coiffage »*, une composition capillaire destinée au maintien et/ou à la fixation de la coiffure.

On connaît déjà des produits cosmétiques capillaires procurant du gonflant au cheveux. Ces produits, à base de polymères filmogènes, sont particulièrement appréciés par les utilisateurs ayant des cheveux fins.

Toutefois, une de leurs limites réside dans le fait que le gonflant disparaît dès le premier shampooing. De plus, ces produits laissent un toucher rêche, qui résulte du dépôt de polymères à la surface des cheveux.

Pour donner du gonflant aux cheveux, il est également possible d'effectuer des permanentes. Ces dernières, basées sur l'utilisation de réducteurs et d'oxydants, nécessitent une mise sous tension mécanique des cheveux, par exemple au moyen de rouleaux. Si de tels traitements ont effectivement pour effet d'augmenter le volume des cheveux, ils peuvent néanmoins présenter l'inconvénient d'altérer l'état de la fibre capillaire.

Le document US 5 830 509 décrit des compostions à usage pharmaceutique contenant des microparticules d'alumine. Le document US 3 819 827 décrit des compostions capillaires contenant de l'alumine ou de la silice finement divisé.

Le problème posé par l'invention est de trouver des compositions cosmétiques capillaires qui procurent aux cheveux du gonflant, sans présenter l'ensemble des inconvénients énumérés ci-dessus.

De manière surprenante et inattendue, la Demanderesse a découvert qu'il était possible de résoudre ce problème, en utilisant des nanoparticules contenant de l'alumine dans un milieu cosmétiquement acceptable.

L'invention a pour objet une composition cosmétique, notamment capillaire, caractérisée par le fait qu'elle comprend, dans un milieu cosmétiquement acceptable, des nanoparticules contenant au moins 10 % d'alumine.

Un autre objet de l'invention concerne un procédé cosmétique capillaire mettant en oeuvre cette composition.

Encore un autre objet de l'invention concerne l'utilisation de nanoparticules contenant de l'alumine pour donner du gonflant aux cheveux ou aider au maintien de la coiffure.

On entend par *« nanoparticules* », des particules solides de taille primaire moyenne en nombre comprise entre 5 et 25 nm, et de préférence entre 8 et 18 nm.

Conformément à la présente invention, les particules solides contenant de l'alumine forment une masse dans laquelle l'alumine ne sert pas d'agent de revêtement, d'enrobage, ou d'adsorption à une (ou d'autres) charge(s), comme un ou (des) oxyde(s) métallique(s).

Dans le cas où les nanoparticules sont formées par de l'alumine et d'autres charges, l'alumine se trouve à l'état libre et ne forme pas de liaisons chimiques avec les autres charges. Il s'agit alors d'un alliage entre l'alumine et d'autres charges, notamment avec des oxydes de métaux ou de métalloïdes, en particulier obtenu par fusion thermique de ces différents constituants.

Au sens de la présente invention, on entend par "taille primaire de particule", la dimension maximale qu'il est possible de mesurer entre deux points diamétralement opposés de la particule. La taille primaire moyenne en nombre est déterminable soit par microscopie électronique à transmission, soit par mesure de la surface spécifique par la technique BET.

Les particules selon l'invention peuvent par exemple avoir une forme quelconque, par exemple la forme de sphères, de paillettes, d'aiguilles ou des formes totalement aléatoires.

Lorsque les nanoparticules contenant au moins 10 % d'alumine comprennent en outre un oxyde de métal ou de métalloïde autre que l'alumine, celui ci est notamment choisi parmi l'oxyde de silicium ou de bore.

Plus préférentiellement encore, les nanoparticules contiennent au moins 50 % d'alumine.

Selon un mode plus avantageux de la présente invention, les nanoparticules sont des nanoparticules d'alumine.

Avantageusement, les nanoparticules contenant de l'alumine sont présentes dans la composition à une concentration, en poids par rapport au poids total de la composition, comprise entre 0,01 % et 30 %, et de préférence entre 0,05 % et 5 %.

Plus avantageusement encore, les compositions capillaires conformes à l'invention contiennent, en outre, au moins un agent activateur. On entend par *" agent activateur "*, tout constituant capable de favoriser l'absorption d'eau par les cheveux et/ou d'augmenter la quantité d'eau qu'ils absorbent. En particulier, l'agent activateur est choisi dans le groupe comprenant :
(i) les alcanes polyols, tels que le glycérol, le propylène glycol et l'héxylène 1,2 diol,
(ii) les bases azotées, telles que l'ammoniaque, les amines primaires, secondaires ou tertiaires, les sels organiques ou minéraux de guanidine, l'urée et ses dérivés N-alkylés et leurs mélanges.

Conformément à l'invention, les nanoparticules contenant de l'alumine peuvent aussi être stockées séparément de la composition cosmétique capillaire comprenant éventuellement l'agent activateur, les nanoparticules contenant de l'alumine étant introduites dans la composition cosmétique capillaire au moment de l'emploi. Dans ce cas, il est proposé, par exemple, un kit ayant au moins deux compartiments, l'un au moins des compartiments comprenant des nanoparticules contenant de l'alumine.

Avantageusement, la concentration en poids par rapport au poids total de la composition, en agent activateur est comprise entre 0,1 % et 50 %, et de préférence entre 0,3% et 15%. En particulier, le rapport en poids des nanoparticules contenant de l'alumine à l'agent activateur est inférieur à 1, plus préférentiellement inférieur à 0,5 ; et plus préférentiellement encore inférieur à 0,2.

De préférence, les nanoparticules contenant de l'alumine sont utilisées à l'état natif, c'est-à-dire sans être enrobées ou revêtues par un agent quelconque, comme un polymère.

Les compositions de l'invention peuvent également comprendre d'autres type de nanoparticules, comme des nanoparticules d'oxydes de titane ou de zinc.

De façon préférée, la composition cosmétique capillaire comprend au moins deux agents activateurs, en particulier choisis parmi ceux énumérés précédemment.

Avantageusement, selon le procédé conforme à l'invention:
- On applique sur cheveux secs ou humides la composition conforme à l'invention,
- On laisse sécher naturellement les cheveux ou par application d'une source de chaleur
- On rince, éventuellement, les cheveux après l'application de la composition, dans ce cas le temps de pose de la composition avant rinçage est compris entre 2 minutes et 1 heure,
- Après rinçage, on laisse sécher naturellement les cheveux ou par application d'une source de chaleur.

Les compositions conformes à l'invention contiennent, de préférence de l'eau et/ou un solvant organique choisi dans le groupe comprenant les alcools en C₁ à C₄, tels que l'éthanol ou l'isopropanol, les alcanes en C₅ à C₁₀, l'acétone, la méthyléthylcétone, l'acétate de méthyle, l'acétate de butyle, l'acétate d'éthyle, le diméthoxyéthane, le diéthoxyéthane et leurs mélanges.

Un mode particulier de l'invention consiste à employer des compositions contenant outre les nanoparticules contenant de l'alumine, l'alcool éthylique comme milieu de dispersion principal. Dans ce cas, le taux d'alcool éthylique par rapport à l'ensemble des solvants, est d'au moins 50%.

Avec ces compositions à majorité alcoolique, le procédé d'utilisation préféré est le suivant :
- on applique sur cheveux secs la composition conforme à l'invention,
- on laisse sécher naturellement les cheveux ou par application d'une source de chaleur.

Elles peuvent contenir, en outre, des additifs cosmétiques usuels.

Les compositions conformes à l'invention peuvent être conditionnées sous diverses forme. Elles peuvent être utilisées en application rincée ou non.

Les compositions conformes à l'invention peuvent être utilisées en tant que compositions de fixation et/ou de maintien des cheveux, compositions de soin de cheveux, shampooings, compositions de conditionnement des cheveux, telles que des compositions destinées à apporter de la douceur aux cheveux, ou encore des compositions de maquillage des cheveux.

Afin d'évaluer le gonflant des cheveux, on met en oeuvre le test décrit ci-après, et appelé *« test du pendule de souplesse »*. Cette méthode repose sur la détermination de la rigidité en flexion des cheveux après l'application d'une composition conforme à l'invention sur des cheveux eurochâtains lavés puis séchés.

Le dispositif est formé par un socle horizontal, une tige métallique verticale fixe de 30 centimètres de long, un balancier de 18,5 cm, et une masse pesante en laiton se présentant sous la forme d'une barre cylindrique et fixée à l'extrémité libre du balancier La barre en laiton pèse 47 grammes et mesure 5 centimètres de longueur.

On prépare des éprouvettes de cheveux se présentant sous la forme d'un peigne. Pour cela, on dispose 39 morceaux de cheveux de 11 mm de long perpendiculairement au bord d'une plaque rectangulaire métallique de 15,5 grammes, et de dimension 40mm x 15mmx9mm.

On applique la composition conforme à l'invention directement sur les cheveux déjà disposés sur les éprouvettes. Les mesures ainsi que le séchage des éprouvettes de cheveux s'effectuent à température et humidité relative contrôlées (20°C et 45% HR).

On dispose successivement chaque éprouvette de cheveux en-dessous du balancier En position de repos, la barre en laiton est en contact avec l'extrémité libre des cheveux.

On déplace à la main la barre en laiton jusqu'à ce que l'angle entre la verticale et le balancier soit égal à 30°. On lâche la barre en laiton sans vitesse initiale. A chaque passage à la verticale du balancier, la barre vient en contact avec l'extrémité des cheveux. Selon la rigidité des cheveux, on estime que ceux-ci ralentissent plus ou moins le mouvement du balancier et que le temps nécessaire à l'arrêt total du balancier est représentatif de la vigueur des cheveux, et de leur ressort.

On note donc, pour chaque éprouvette, le temps nécessaire à l'arrêt total du balancier ou, de façon équivalente, le nombres de battements du balancier avant son arrêt total. On compare les résultats obtenus pour chacune des éprouvettes. On considère que moins le balancier met de temps à s'arrêter, plus les cheveux ont du ressort et de la vigueur.

L'invention pourra être mieux comprise à l'aide des exemples non limitatifs qui suivent et qui constituent des modes de mise en oeuvre préférentiels du procédé conforme à l'invention.

Dans les exemples, les pourcentages sont exprimés en poids et m. a. signifie matière active.

### EXEMPLE COMPARATIF :

On réalise les compositions suivantes :

| Composition A (invention) : | |
|---|---|
| Nanoparticules d'alumine ⁽¹⁾ | 0,5 % |
| Eau | 99,5 % |
| | |

| Témoin | |
|---|---|
| Eau | 100 % |

| | |
|---|---|
| (1) Nanoparticules présentant une taille de particule primaire moyenne en nombre de 13 nm, commercialisées sous la dénomination ALUMINIUMOXYD C par la Société DEGUSSA-HULS. | |

On utilise le test du *« pendule de souplesse »* pour évaluer le gonflant des cheveux traités par la composition A.

Des cheveux naturels eurochâtains collés sur une éprouvette sont trempés dans le témoin pendant 15 minutes, puis l'éprouvette est séchée pendant 12 heures dans les conditions, à savoir 20°C et 45% HR. Après séchage, on mesure le nombre de battements nécessaire à l'arrête du pendule. Les cheveux de la même éprouvette sont ensuite trempés pendant 15 minutes dans la composition A, puis on effectue le séchage des cheveux et la mesure d'amortissement du pendule dans les mêmes conditions que celles définies pour le témoin.

10 éprouvettes successivement traitées par le témoin puis par la composition A sont testées, puis la moyenne des écarts du nombre de battements avant et après traitement est calculée

On observe que le traitement des cheveux par les nanoparticules d'alumine a pour effet de diminuer, en moyenne, de 10 % ± 2 % le nombre de battements nécessaire a l'arrêt du pendule, par rapport au témoin.

Il en résulte que les compositions conformes à l'invention comprenant des nanoparticules d'alumine confèrent aux cheveux de la vigueur et du ressort. Sur la tête d'une personne, cette effet se traduit par une augmentation du volume global de sa coiffure.

On évalue également par un test sensoriel la douceur des cheveux après application de la composition. A. Pour ceci, on utilise 10 modèles possédant des cheveux naturels châtains mi-longs. Sur 5 de ces modèles, via un spray, on pulvérise 4g de composition A et sur les 5 autres modèles on pulvérise 4 grammes de témoin, puis on laisse sécher naturellement les cheveux.

Les chevelures des modèles sont évaluées par 10 testeurs expérimentés. Ils estiment, à l'unanimité, que les cheveux traités par les compositions conformes à l'invention ont un toucher plus agréable et sont plus doux que ceux traités par la composition témoin.

Par conséquent, les compositions conformes à l'invention confèrent aux cheveux du gonflant, tout en leur procurant un toucher agréable.

### EXEMPLE ILLUSTRATIF,:

On réalise la composition suivante :

| Composition B (invention) : | |
|---|---|
| Nanoparticules d'alumine ⁽¹⁾ | 0,5 % |
| Alcool éthylique | 99,5 % |
| | |

| Témoin | |
|---|---|
| Alcool éthylique | 100 % |

| | |
|---|---|
| (1) Nanoparticules présentant une taille de particule primaire moyenne en nombre de 13 nm, commercialisées sous la dénomination ALUMINIUMOXYD C par la Société DEGUSSA-HULS. | |

On évalue par un test sensoriel la fixation et la douceur de chevelures après application de la composition B. Pour ceci, on utilise 10 modèles possédant des cheveux naturels châtains mi-longs. Sur 5 de ces modèles, via un spray, on pulvérise 4g de composition B et sur les 5 autres modèles on pulvérise 4 grammes de témoin, puis on laisse sécher naturellement les cheveux.

Les chevelures des 10 modèles sont évaluées par 10 testeurs expérimentés. Ils estiment, à l'unanimité, que les cheveux traités par la composition B ont un toucher plus agréable, plus doux, sont plus brillants et possèdent un meilleur maintien que ceux traités par la composition témoin.

## Revendications

1. Composition cosmétique, notamment capillaire, **caractérisée par le fait qu'**elle comprend, dans un milieu cosmétiquement acceptable, des nanoparticules contenant au moins 10 % d'alumine de taille primaire moyenne en nombre comprise entre 5 et 25 nm.

2. Composition selon la revendication 1, **caractérisée par le fait que** les nanoparticules contenant au moins 10 % d'alumine comprennent, en outre, un oxyde de métal ou de métalloïde autre que l'alumine, et notamment choisi parmi l'oxyde de silicium ou de bore.

3. Composition selon l'une quelconque des revendications précédentes, **caractérisée par le fait que** les nanoparticules contiennent au moins 50 % d'alumine.

4. Composition selon la revendication 1, **caractérisée par le fait que** les nanoparticules sont des nanoparticules d'alumine.

5. Composition selon l'une quelconque des revendications précédentes, **caractérisée par le fait que** la taille primaire moyenne en nombre des particules est comprise entre 8 et 18 nm.

6. Composition selon l'une quelconque des revendications précédentes, **caractérisée par le fait que** les nanoparticules contenant de l'alumine sont présentes dans la composition à une concentration, en poids par rapport au poids total de la composition, comprise entre 0,01 % et 30 %, et de préférence entre 0,05 % et 5 %.

7. Composition selon l'une quelconque des revendications précédentes, **caractérisée par le fait qu'**elle comprend, en outre, au moins un agent activateur, choisi dans le groupe comprenant :
(i) les alcanes polyols, tels que notamment le glycérol, le propylène glycol et l'héxylène 1,2 diol,
(ii) les bases azotées, telles que l'ammoniaque, les amines primaires, secondaires ou tertiaires, les sels organiques ou minéraux de guanidine, l'urée et ses dérivés N-alkylés et leurs mélanges.

8. Composition selon la revendication 7, **caractérisée par le fait que** la concentration en poids par rapport au poids total de la composition, en agent activateur est comprise entre 0,1 % et 50 %, et de préférence entre 0,3 % et 15 %.

9. Composition selon l'une quelconque des revendications 7 ou 8, **caractérisée par le fait que** le rapport en poids des nanoparticules contenant de l'alumine à l'agent activateur est inférieur à 1, plus préférentiellement inférieur à 0,5 ; et plus préférentiellement encore inférieur à 0,2.

10. Composition selon l'une quelconque des revendications précédentes, **caractérisée par le fait qu'**elle comprend de l'eau et/ou un solvant organique choisi dans le groupe comprenant les alcools en C₁ à C₄, tels que l'éthanol ou l'isopropanol, les alcanes en C₅ à C₁₀, l'acétone, la méthyléthylcétone, l'acétate de méthyle, l'acétate de butyle, l'acétate d'éthyle, le diméthoxyéthane, le diéthoxyéthane et leurs mélanges.

11. Kit ayant au moins deux compartiments, **caractérisé par le fait qu'**au moins l'un des compartiments comprend des nanoparticules contenant de l'alumine de taille primaire moyenne en nombre comprise entre 5 et 25 nm.

12. Procédé cosmétique capillaire, **caractérisé par le fait que** :
- On applique sur cheveux secs ou humides la composition conforme à l'invention selon les revendications 1 à 11,
- On laisse sécher naturellement les cheveux ou par application d'une source de chaleur
- On rince, éventuellement, les cheveux après l'application de la composition, dans ce cas le temps de pose de la composition avant rinçage est compris entre 2 minutes et 1 heure,
- Après rinçage, on laisse sécher naturellement les cheveux ou par application d'une source de chaleur.

13. Utilisation de nanoparticules contenant de l'alumine présentant une taille primaire moyenne en nombre comprise entre 5 et 25 nm, dans la préparation de compositions capillaires, dans le but d'apporter du gonflant aux cheveux.

14. Utilisation selon la revendication 13, **caractérisée par le fait que** les nanoparticules présentent une taille primaire moyenne en nombre comprise entre 5 et 25 nm.

## Claims

1. Cosmetic composition, in particular hair composition, **characterized in that** it comprises, in a cosmetically acceptable medium, nanoparticles containing at least 10% alumina with a number-average primary size of between 5 nm and 25 nm.

2. Composition according to Claim 1, **characterized in that** the nanoparticles containing at least 10% alumina also comprise a metal oxide or metalloid oxide other than alumina, and chosen in particular from silicon oxide and boron oxide.

3. Composition according to either of the preceding claims, **characterized in that** the nanoparticles contain at least 50% alumina.

4. Composition according to Claim 1, **characterized in that** the nanoparticles are alumina nanoparticles.

5. Composition according to any one of the preceding claims, **characterized in that** the number-average primary particle size is between 8 nm and 18 nm.

6. Composition according to any one of the preceding claims, **characterized in that** the nanoparticles containing alumina are present in the composition at a concentration, by weight relative to the total weight of the composition, of between 0.01% and 30% and preferably between 0.05% and 5%.

7. Composition according to any one of the preceding claims, **characterized in that** it also comprises at least one activator chosen from the group comprising:
(i) alkane polyols, in particular such as glycerol, propylene glycol and hexylene-1,2-diol,
(ii) nitrogenous bases, such as aqueous ammonia, primary, secondary or tertiary amines, organic or inorganic guanidine salts, urea and its N-alkyl derivatives, and mixtures thereof.

8. Composition according to Claim 7, **characterized in that** the concentration by weight, relative to the total weight of the composition, of activator is between 0.1% and 50% and preferably between 0.3% and 15%.

9. Composition according to either of Claims 7 and 8, **characterized in that** the weight ratio of the nanoparticles containing alumina to the activator is less than 1, more preferably less than 0.5; and even more preferably less than 0.2.

10. Composition according to any one of the preceding claims, **characterized in that** it comprises water and/or an organic solvent chosen from the group comprising C₁ to C₄ alcohols, such as ethanol or isopropanol, C₅ to C₁₀ alkanes, acetone, methyl ethyl ketone, methyl acetate, butyl acetate, ethyl acetate, dimethoxyethane and diethoxyethane, and mixtures thereof.

11. Kit having at least two compartments, **characterized in that** at least one of the compartments comprises nanoparticles containing alumina with a number-average primary size of between 5 nm and 25 nm.

12. Cosmetic hair process, **characterized in that**:
- the composition in accordance with the invention according to Claims 1 to 11 is applied to dry or wet hair,
- the hair is allowed to dry naturally or by applying a source of heat,
- after applying the composition, the hair is optionally rinsed, and in this case the time for which the composition is left to stand on the hair before rinsing out is between 2 minutes and 1 hour,
- after rinsing, the hair is left to dry naturally or by applying a source of heat.

13. Use of nanoparticles containing alumina, with a number-average primary size of between 5 nm and 25 nm, in the preparation of hair compositions, with the aim of giving the hair body.

14. Use according to Claim 13, **characterized in that** the nanoparticles have a number-average primary size of between 5 nm and 25 nm.

## Patentansprüche

1. Kosmetische Zusammensetzung, insbesondere für die Haarbehandlung, **dadurch gekennzeichnet, dass** sie in einem kosmetisch akzeptablen Medium Nanopartikel enthält, die mindestens 10 % Aluminiumoxid mit einer zahlenmittleren Primärgröße im Bereich von 5 bis 25 nm enthalten.

2. Zusammensetzung nach Anspruch 1, **dadurch gekennzeichnet, dass** die Nanopartikel, die mindestens 10 % Aluminiumoxid enthalten, ferner ein von Aluminiumoxid verschiedenes Metalloxid oder Halbmetalloxid enthalten, das insbesondere unter Siliciumoxid oder Boroxid ausgewählt ist.

3. Zusammensetzung nach einem der vorhergehenden Ansprüche, **dadurch gekennzeichnet, dass** die Nanopartikel mindestens 50 % Aluminiumoxid enthalten.

4. Zusammensetzung nach Anspruch 1, **dadurch gekennzeichnet, dass** die Nanopartikel Aluminiumoxid-Nanopartikel sind.

5. Zusammensetzung nach einem der vorhergehenden Ansprüche, **dadurch gekennzeichnet, dass** die zahlenmittlere Primärgröße der Partikel im Bereich von 8 bis 18 nm liegt.

6. Zusammensetzung nach einem der vorhergehenden Ansprüche, **dadurch gekennzeichnet, dass** die Aluminiumoxid enthaltenden Nanopartikel in der Zusammensetzung in einer Konzentration von 0,01 bis 30 Gew.-% und vorzugsweise 0,05 bis 5 Gew.-%, bezogen auf das Gesamtgewicht der Zusammensetzung, enthalten sind.

7. Zusammensetzung nach einem der vorhergehenden Ansprüche, **dadurch gekennzeichnet, dass** sie ferner mindestens einen Aktivator enthält, der ausgewählt ist unter:
(i) Alkanpolyolen, insbesondere Glycerin, Propylenglycol und Hexylen-1,2-diol,
(ii) stickstoffhaltigen Basen, wie Ammoniak, primären, sekundären oder tertiären Aminen, organischen oder anorganischen Guanidinsalzen, Harnstoff und seinen N-alkylierten Derivaten und deren Gemischen.

8. Zusammensetzung nach Anspruch 7, **dadurch gekennzeichnet, dass** die Konzentration des Aktivators im Bereich von 0,1 bis 50 Gew.-% und vorzugsweise 0,3 bis 15 Gew.-%, bezogen auf das Gesamtgewicht der Zusammensetzung, liegt.

9. Zusammensetzung nach einem der Ansprüche 7 oder 8, **dadurch gekennzeichnet, dass** das Gewichtsverhältnis der Aluminiumoxid enthaltenden Nanopartikel und des Aktovators unter 1, vorzugsweise unter 0,5 und noch bevorzugter unter 0,2 liegt.

10. Zusammensetzung nach einem der vorhergehenden Ansprüche, **dadurch gekennzeichnet, dass** sie Wasser und/oder ein organisches Lösungsmittel enthält, das unter den C₁₋₄-Alkoholen, wie Ethanol oder Isopropanol, C₅₋₁₀-Alkanen, Aceton, Methylethylketon, Methylacetat, Butylacetat, Ethylacetat, Dimethoxyethan, Diethoxyethan und deren Gemischen ausgewählt ist.

11. Kit mit mindestens zwei Abteilungen, **dadurch gekennzeichnet, dass** mindestens eine Abteilung Aluminiumoxid enthaltende Nanopartikel mit einer zahlenmittleren Primärgröße von 5 bis 25 nm enthält.

12. Kosmetisches Verfahren für die Haarbehandlung, **dadurch gekennzeichnet, dass**:
- auf die trockenen oder feuchten Haare die erfindungsgemäße Zusammensetzung nach den Ansprüchen bis 11 aufgetragen wird,
- die Haare auf natürliche Weise trocknen gelassen oder durch Verwendung einer Wärmequelle getrocknet werden,
- die Haare nach dem aufbringen der Zusammensetzung gegebenenfalls gespült werden, wobei in diesem Fall die Einwirkzeit der Zusammensetzung vor dem Spülen im Bereich von 2 Minuten bis 1 Stunde liegt,
- die Haare nach dem Trocknen auf natürliche Weise trocknen gelassen oder durch Verwendung einer Wärmequelle getrocknet werden.

13. Verwendung von Aluminiumoxid enthaltenden Nanopartikeln, die eine zahlenmittlere Primärgröße von 5 bis 25 nm aufweisen, für die Herstellung von Zusammensetzungen für die Haarbehandlung, um dem Haar Volumen zu geben.

14. Verwendung nach Anspruch 13, **dadurch gekennzeichnet, dass** die Nanopartikel eine zahlenmittlere Primärgröße von 5 bis 25 nm aufweisen.
